# EUROPEAN PATENT SPECIFICATION

(11) **EP 1 008 242 B1**
(45) Date of publication and mention of the grant of the patent: **05.11.2003**
(21) Application number: 98944155.5
(22) Date of filing: 20.08.1998
(51) Int. Cl.: G01N 33/50

(54) **USE OF SHP-1 AND SHP-2 TO DETECT COMPOUNDS INVOLVED IN NEURONAL SURVIVAL**
SHP-1 UND SHP-2 ALS MARKER FÜR APOPTOSE-INDUZIERENDE VERBINDUNGEN IN NEURONEN
UTILISATION DE SHP-1 ET SHP-2 POUR DECELER DES COMPOSES IMPLIQUES DANS LA SURVIE NEURONALE

(30) Priority: 25.08.1997 US 918157
(43) Date of publication of application: 14.06.2000
(73) Proprietor: McGILL UNIVERSITY, Montreal, Québéc H3A 2T5 (CA)
(72) Inventor: KAPLAN, David, Montreal, Quebec H4A 3B3 (CA); MARSH, H., Nick, Montreal, Quebec H2V 4K2 (CA)
(74) Representative: Grund, Martin, Dr.
(86) International application number: IB9801526
(87) International publication number: WO99010998

(56) References cited:
- WO-A-97/10349
- WO-A-98/22608
- BEDECS, KATARINA ET AL: "Angiotensin II type 2 receptors mediate inhibition of mitogen-activated protein kinase cascade and functional activation of SHP - 1 tyrosine phosphatase" BIOCHEM. J. (1997), 325(2), 449-454 CODEN: BIJOAK;ISSN: 0264-6021, 15 July 1997 (1997-07-15), XP002094565
- VAMBUTAS, VIDA ET AL: "Nerve Growth Factor Stimulates Tyrosine Phosphorylation and Activation of Src Homology-containing Protein-tyrosine Phosphatase 1 in PC12 Cells." JOURNAL OF BIOLOGICAL CHEMISTRY, (1995) VOL. 270, NO. 43, PP. 25629-25633 ISSN: 0021-9258., XP002094566
- MARSH, H. N. ET AL.: "Involvement of SHPs in Trk Signaling" SOCIETY FOR NEUROSCIENCE ABSTRACTS (1997), MEETING INFO: 27TH ANNUAL MEETING OF THE SOCIETY FOR NEUROSCIENCE, NEW ORLEANS, LOUSIANA, USA, vol. 23, no. 1-2, 25 - 30 October 1997, page 1434 XP002094427
- YAMADA ET AL., "Angiotensin II type 2 receptor mediates programmed cell death", PROC. NATL. ACAD. SCI. (1996) VOL. 93, PP.156-160

## Description

### Field of the Invention

The field of the invention is cell death.

### Background of the Invention

The development of strategies to promote repair of the degenerating or traumatized nervous system is a major ongoing therapeutic challenge. Current strategies to treat the injured nervous system include transplantation surgery and treatments utilizing recombinant endogenous proteins, such as growth factors. Both of these therapies have significant limitations; the surgical approaches are expensive, invasive and labor-intensive, while growth factors generally cannot cross the blood-brain barrier and often have pleiotropic biological activities.

The survival and regeneration of many injured nerve populations depends upon the action of neurotrophic factors. The prototypical neurotrophin, nerve growth factor (NGF), is necessary for the development, growth, survival, and regeneration of sympathetic neurons, and for the development and rescue from trauma of basal forebrain cholinergic neurons. The neurotrophin family, which also includes brain-derived neurotrophic factor (BDNF), neurotrophin-3 (NT-3), NT-4 and NT-6, together with their receptors, TrkA, TrkB and TrkC, mediate the development and survival of subpopulations of peripheral nervous system (PNS) and central nervous system (CNS) neurons. Upon ligand binding, the Trks undergo autophosphorylation on tyrosine residues, specifically 490, 670, 674, 675, and 785 for human TrkA. Tyrosine phosphorylation of the Trks has two major functions. First, it couples Trk to intracellular signal transduction pathways by allowing Trk to recognize and bind certain cytoplasmic proteins. A second function of ligand-induced Trk tyrosine phosphorylation is to stimulate the catalytic activity of the receptor. Thus, tyrosine phosphorylation of the Trks is critical for the regulation of their neurotrophic activities.

In all cell types, including neurons, the level of tyrosine phosphorylation is regulated by the coordinated actions of both protein-tyrosine kinases (PTKs) and protein-tyrosine phosphatases (PTPs). Previously, we have identified a cytoplasmic PTP that is stimulated by NGF and binds directly to activated TrkA in PC12 pheochromocytoma cells. The PTP, SHP-1, is a member of a family of nontransmembrane PTPs containing SH2 domains (SHPs). A second PTP, SHP-2, is activated by BDNF in cortical neurons. SHP-1 is expressed at highest levels in hematopoietic cells and SHP-2 is expressed ubiquitously. Depending upon the signaling pathway, the SHPs may have both positive and negative signaling roles.

Bedecs K. et al. (1997, Biochem. J. 325, 449-454) describe inhibition of mitogen-activated protein kinase cacade mediated by angiotensin II type 2 receptors. It is disclosed that angiotensin II stimulates the catalytic activity of SHP-1 in N1E-115 and Chinese hamster ovary cells expressing recombinant human angiotensin II type 2 receptors. Furthermore, Yamada et al. (1996) Proc. Natl. Trad. Sci. 93 : 156- 160 describes that PLW12 cells treated with angiotensin II undergo programmed cell death.

WO 98/22608 published on 28.05. 1998 discloses the use of a SHP-containing adenovirus construct for use in an assay for identifying a substance which inhibits/induces synoptosis.

Chronic infusion of neurotrophins is used as an experimental treatment for various neurodegenerative diseases. However, it has been shown that neurotrophin receptors (such as Trks) are downregulated in response to continued exposure to certain heurotrophins. It would be highly desirable to have a means for identifying compounds that prevent attenuation of the neurotrophin signal as such compounds could be used to prevent nerve cell death.

### Summary of the Invention

Here we present evidence *in vitro* and *in vivo* that the SHPs are activated by neurotrophin treatment, that they dephosphorylate Trk, and that they can regulate neuronal survival. SHP-1 and SHP-2 activity is rapidly induced by NGF, as measured by increased association of SHP-2 with cellular tyrosine phosphorylated proteins. *In vivo,* SHP- 1 dephosphorylates Trk very efficiently at phosphotyrosines 674 and 675, residues that we have identified as critical for the regulation of Trk catalytic activity. Trk is dephosphorylated, and therefore inactivated, by both SHP-1 and SHP-2 *in vitro.* Furthermore, the expression of WT and dominant-activated (ACT) forms of SHP-2 promote cell death in cultured sympathetic neurons. SHP-2-mediated cell death is suppressed by the expression of Bcl-2, a protein that has been demonstrated to promote neuronal survival. Taken together, or results show an important role for the SHPs in the regulation of Trk activity and define these PTPs as prime targets for drug targeting by small molecules. The invention provides a means of identifying compounds which modulate SHP biological activity in neurons and which thus regulate neuronal cell death.

The invention features methods of identifying compounds that modulate cell death in a neuron as defined in the claims. In general terms, the method includes the steps of: (a) exposing a population of cells to a test compound; (b) over expressing a SHP polypeptide in said cell; and (c) assaying for an alteration in the level of SHP biological activity, an alteration in the level of SHP biological activity indicating a compound that modulates cell death in a neuron. In various embodiments of this aspect, the cell is induced to express the SHP; the cell is exposed to said test compound before the cell is induced to produce SHP polypeptide; the cell is genetically engineered to express SHP polypeptide; the cell is exposed to said test compound after said cell is induced to express increased levels of the SHP polypeptide.

In preferred embodiments of the above aspects of the invention, SHP is dominant-activated SHP; the SHP biological activity is measured by measuring SHP phosphorylation, SHP interaction with Trk or SHP polypeptide levels.

In various other preferred embodiments of the above aspects of the invention the Trk is Trk A, Trk B, or Trk C; the Trk is phosphorylated; the SHP is SHP-1 or SHP-2; the phosphorylated protein is phosphorylated on one or more tyrosine residues; the neuron is selected from the group consisting of: a sympathetic neuron, a cortical neuron, a hippocampal neuron, a motor neuron, an SCG neuron, a sensory neuron, a dopaminergic neuron; and the neurotrophin is selected from the group consisting of: nerve growth factor (NGF), brain-derived neurotrophic factor, (BDNF), neurotrophin (NT-3), NT-4, and NT-6.

By "apoptosis" is meant the process of cell death wherein a dying cell displays a set of well-characterized biochemical hallmarks which include cytolemmal membrane blebbing, cell soma shrinkage, chromatin condensation, and DNA laddering.

By "neuron" is meant a cell of ectodermal embryonic origin derived from any part of the nervous system of an animal. Neurons express well-characterized neuron-specific markers which include neurofilament proteins, MAP2, and class m β-tubulin. Included as neurons are, for example, hippocampal, cortical, midbrain dopaminergic, motor, sensory, sympathetic, septal cholinergic, and cerebellar neurons.

By "expose" is meant to allow contact between an animal, cell, lysate or extract derived from a cell, or molecule derived from a cell, and a test compound or apoptotic stimulus.

By "treat" is meant to submit or subject an animal, cell, lysate or extract derived from a cell, or molecule derived from a cell to a test compound or apoptotic stimulus.

By "test compound" is meant a chemical, be it naturally-occurring or artificially-derived, that is surveyed for its ability to modulate cell death, by employing one of the assay methods described herein. Test compounds may include, for example, peptides, polypeptides, synthesized organic molecules, naturally occurring organic molecules, nucleic acid molecules, and components thereof.

By "assaying" is meant analyzing the effect of a treatment or exposure, be it chemical or physical, administered to whole animals or cells derived therefrom. The material being analyzed may be an animal, a cell, a lysate or extract derived from a cell, or a molecule derived from a cell. The analysis may be, for example, for the purpose of detecting altered gene expression, altered nucleic acid stability (e.g. mRNA stability), altered protein stability, altered protein levels, or altered protein biological activity. The means for analyzing may include, for example, nucleic acid amplification techniques, reporter gene assays, antibody labeling, immunoprecipitation, and phosphorylation assays and other techniques known in the art for conducting the analysis of the invention.

By "modulating" is meant changing, either by decrease or increase.

By "a decrease" is meant a lowering in the level of protein, or protein phosphorylation, of at least 3-fold as measured, for example, by ELISA.

By "an increase" is meant a rise in the level of protein, or protein phosphorylation, of at least 3-fold as measured, for example, by ELISA.

By "protein" or "polypeptide" or "polypeptide fragment" is meant any chain of more than two amino acids, regardless of post-translational modification (e.g., glycosylation or phosphorylation), constituting all or part of a naturally-occurring polypeptide or peptide, or constituting a non-naturally occurring polypeptide or peptide.

By "SHP polypeptide" is meant a protein-tyrosine phosphatase having an SH2 domain and expressed in neuronal cells. Preferably, the SHP is SHP-1 or SHP-2, most preferably the SHP is the SHP-1 provided in Genbank, Accession Number M77273 or the SHP-2 provided in Genbank, Accession Number L03535.

By "promoter" is meant a minimal sequence sufficient to direct transcription of an operably-linked gene.

By "SH2 domain" is meant a polypeptide domain of about 100 amino acid residues which has structural and functional identity (i.e., the ability to physically interact with phosphotyrosine residues) to the second homology domain of the src tyrosine kinase.

By "alteration in the level of cell death" is meant increasing or decreasing the number of cells that undergo apoptotic cell death (than would otherwise be the case) in a given cell population. Preferably, the cell population is selected from a group including neuronal cells, fibroblasts, baculovirus-infected insect cells, or any other type of primary cell or established cell line known to undergo apoptotic cell death either *in vivo* or *in vitro.* It will be appreciated that the degree of cell death in a given assay will vary, but that one skilled in the art can determine the statistically significant change or a therapeutically effective change in the level of cell death which identifies a compound which modulates SHP biological activity and hence, modulates cell death. Preferably the alteration is a change of at least 10%, more preferably 30%, and most preferably 2-fold.

### Brief Description of the Drawings

Fig. 1 is a Western blot showing that SHP-1 and SHP-2 are expressed in PC12 cells and sympathetic neurons.
Fig. 2 is a Western blot showing that NGF stimulates tyrosine phosphorylation of SHP-1 in PC12 cells. Lysates prepared from cells treated with NGF were immunoprecipitated with anti-SHP-1 and probed with an anti-phosphotyrosine (pTyr) antibody (top panel).
Fig. 3 is a Western blot showing that NGF stimulates the association of SHP-2 with tyrosine-phosphorylated proteins in PC12 cells. Lysates prepared from PC12 cells treated with NGF for the indicated times were immunoprecipitated with anti-SHP-2 and probed with anti-pTyr antibodies.
Fig. 4 is a Western blot showing that NGF stimulates an increased association of tyrosine-phosphorylated proteins with SHP-2 in SCG neurons. Lysates prepared from neurotrophin-treated SCGs were immunoprecipitated with anti-SHP-2 antibody and immunoblotted with anti-pTyr. Lanes I and 2: NGF-stimulated association of tyrosine-phosphorylated proteins with SHP-2. Lanes 3 and 4: NGF-stimulated increase in tyrosine phosphorylation of SCG proteins.
Fig. 5 is a Western blot showing that BDNF stimulates the association of SHP-2 with tyrosine phosphorylated proteins in cortical neurons. Lysates prepared from cortical neurons treated with or without BDNF were immunoprecipitated with anti-SHP-2 antibody and probed with anti-pTyr antibody.
Fig. 6 is a graph showing that expression of SHP-2 increases cell death in cultured sympathetic neurons.
Fig. 7 is a Western blot showing that SHP-1 associates with baculovirus-expressed TrkA, TrkB and TrkC. TrkA, B and C were activated and autophosphorylated *in vitro,* incubated with PC12 cell lysates, fractionated by electrophoresis, and probed with anti-SHP-1 (bottom panel), and anti-pan Trk antibodies (upper panel).
Fig. 8 is a Western blot showing that Y490 of TrkA is required for its association with SHP-1. The Trk receptors analyzed were wild-type TrkA (Trk), Flag-tagged Trk (Flag Trk), or receptors containing mutations at Y490, (Y490F), Y785, (Y785F), Y490 and Y785 (Y490/785F) and K538 (K538N).
Fig. 9 is a Western blot showing that SHP-2 does not associate with baculovirus-expressed Trk. Wild-type and mutant TrkA receptors expressed in Sf9 cells were autophosphorylated, incubated with PC12 lysates and immunoblotted with anti-SHP-2.
Fig. 10 is an autoradiogram showing that SHP-1 and SHP-2 dephosphorylate Trk *in vitro*. Trk, SHP-1 and SHP-2 were expressed in Sf9 insect cells and isolated by immunoprecipitation with anti-Trk, anti-SHP-1 or anti-SHP-2 antibody. Immunoprecipitated Trk was autophosphorylated with [γ-^{32p}]ATP and then was incubated with partially purified SHP-1 or SHP-2. Trk phosphorylation levels then were analyzed by SDS/PAGE and autoradiography.
Fig. 11 is a Western blot showing that Y490 and Y785 of TrkA are not required for SHP-1 and SHP-2-mediated dephosphorylation of TrkA *in vivo*. Lysates of Sf9 insect cells coexpressing SHP-1 or SHP-2 wild-type and mutant TrkAs, as indicated, were immunoprecipitated with anti-Trk and probed with anti-pTyr antibody (upper panel) to determine TrkA phosphorylation levels. Lysates were immunoblotted with anti-Trk (second panel), anti-SHP-1 (third panel) or anti-SHP-2 (bottom panel) antibodies to demonstrate that similar amounts of proteins were expressed in the Sf9 cells.
Fig. 12 is a Western blot showing that Y674/Y675 of TrkA are targets of SHP-1 and SHP-2-mediated dephosphorylation. Lysates of Sf9 insect cells co-expressing SHP-1 or SHP-2 and wild-type TrkA using antibodies that specifically recognize these phosphorylated residues. Blots were probed with anti-pan Trk (C,D), anti-SHP-1 (E,F), or anti-SHP2 (G,H) antibodies.

### Description of the Preferred Embodiments

Development of assays for compounds which modulate neuronal apoptosis with high specificity requires, first, identification of key, specific steps in the apoptotic pathway involved and, second, development of assays to measure changes at these steps.

To determine the mechanism whereby the neurotrophins stimulate the survival and growth of normal and injured neurons, we have asked whether the phosphoprotein phosphatases SHP-1 and SHP-2 play a role in the differentiation and survival of primary neurons, focusing our studies on cultured rat sympathetic cervical ganglion (SCG) and cortical neurons in order to determine the exact nature of this regulation. The examples below show that the SHPs, including SHP-1 and SHP-2, are excellent targets for small molecule drugs.

### Primary screens for compounds that modulate SHP activity and neuronal cell death.

Modulations SHP biological activity (which will be demonstrated in the Examples below), modulate neuronal cell death. This finding allows us to provide assays for drugs which modulate neuronal cell death by modulation of SHP biological activity. Such assays may measure SHP biological activity by measuring changes in: (a) SHP phosphorylation status; (b) SHP association with cellular phosphoproteins; (c) levels of SHP-induced neuronal cell death; (d) levels of SHP mRNA or gene expression and in addition; and (e) the *in vivo* and *in vitro* association of SHP with Trk receptors. Such measurements may be made *in vitro* or *in vivo* and form the basis of assays which identify compounds that modulate neuronal cell death. Such identified compounds may have therapeutic value in the treatment of neurodegenerative disease and neurological trauma.

### Secondary screens for compounds that modulate SHP activity and neuronal cell death.

After test compounds that appear to modulate SHP activity are identified, it may be necessary or desirable to subject these compounds to further testing. The invention provides such secondary confirmatory assays. For example, a compound that appears to inhibit SHP activity in early testing will be subject to additional assays to confirm that the compound also inhabits neuronal cell death. Late-stage testing will be performed *in vivo* to confirm that the compounds initially identified to modulate SHP biological activity and death of cultured neurons will have the predicted effect on *in vivo* neurons. In the first round of *in vivo* testing, neuronal cell death is initiated in animals, by well-known methods such as axotomy or cerebral ischemia, and then the compound is administered by one of the means described in the Therapy section immediately below. Neurons or neural tissue are isolated within hours to days following the insult, and are subjected to assays to assess the level of cell death. Such assays are well known to those skilled in the art. Examples of such assays include, but are not limited to: MTT assays, annexin V assays, TUNEL assays, and propidium iodide assays.

### Test Compounds

In general, novel drugs for prevention or treatment of neuronal cell death which functions by targeting SHP biological activity are identified from large libraries of both natural products or synthetic (or semi-synthetic) extracts or chemical libraries according to methods known in the art Those skilled in the field of drug discovery and development will understand that the precise source of test extracts or compounds is not critical to the screening procedure(s) of the invention. Accordingly, virtually any number of chemical extracts or compounds can be screened using the exemplary methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, NH) and Aldrich Chemical (Milwaukee, WI). Alternatively, libraries of natural compounds in the form of bacterial, fungal, plant, and animal extracts are commercially available from a number of sources, including Biotics (Sussex, UK), Xenova (Slough, UK), Harbor Branch Oceangraphics Institute (Ft. Pierce, FL), and PharmaMar, U.S.A. (Cambridge, MA). In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

In addition, those skilled in the art of drug discovery and development readily understand that methods for dereplication (e.g., taxonomic dereplication, biological dereplication, and chemical dereplication, or any combination thereof) or the elimination of replicates or repeats of materials already known for their therapeutic activities for neurodegenerative disorders should be employed whenever possible.

When a crude extract is found to prevent or delay neuronal cell death, further fractionation of the positive lead extract is necessary to isolate chemical constituents responsible for the observed effect. Thus, the goal of the extraction, fractionation, and purification process is the careful characterization and identification of a chemical entity within the crude extract having neuronal cell death-preventative or -palliative activities. The same assays described herein for the detection of activities in mixtures of compounds can be used to purify the active component and to test derivatives thereof. Methods of fractionation and purification of such heterogenous extracts are known in the art. If desired, compounds shown to be useful agents for treatment are chemically modified according to methods known in the art. Compounds identified as being of therapeutic value may be subsequently analyzed using a mammalian neuronal cell death model.

Below are examples of high-throughput systems useful for evaluating the efficacy of a molecule or compound in treating, preventing, or enhancing a neuronal cell death-associated condition.

### Therapy

Compounds identified using any of the methods disclosed herein, may be administered to patients or experimental animals with a pharmaceutically-acceptable diluent, carrier, or excipient, in unit dosage form. Conventional pharmaceutical practice may be employed to provide suitable formulations or compositions to administer such compositions to patients or experimental animals. Although intravenous administration is preferred, any appropriate route of administration may be employed, for example, parenteral, subcutaneous, intramuscular, intracranial, intraorbital, ophthalmic, intraventricular, intracapsular, intraspinal, intracisternal, intraperitoneal, intranasal, aerosol, or oral administration. Therapeutic formulations may be in the form of liquid solutions or suspensions; for oral administration, formulations may be in the form of tablets or capsules; and for intranasal formulations, in the form of powders, nasal drops, or aerosols.

Methods well known in the art for making formulations are found in, for example, "Remington's Pharmaceutical Sciences." Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for antagonists or agonists of the invention include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The following examples are to illustrate the invention. They are not meant to limit the invention in any way.

### EXAMPLE I

### General Methods

### Cells, Growth Factors and Antibodies

PC12 cells are grown in DMEM containing 10% heat-inactivated horse serum and 5% fetal bovine serum. Primary cultures of superior cervical ganglion (SCG) neurons cultures are prepared according to the procedure of Ma et al., J. Cell. Biol., 117:135-141, 1992. Cortical neurons are cultured and maintained as described by Widmer et al., J. Neurochem. 60: 2111-2123, 1993. In addition, methods of culturing these and other types of neurons are well-known to those skilled in the art.

The anti-phosphotyrosine (pTyr) antibody 4G10, anti-SHP-1 and anti-SHP-2 polyclonal antibodies were obtained from Upstate Biotechnology (Lake Placid, NY) and used as directed. SHP-1 monoclonal antibody was from Transduction Laboratories (Lexington, KY). Anti-phosphoTrk antibodies were obtained from New England Biolabs (Beverly, MA). Anti-Trk (203) was used as previously described (Hempstead et al., Neuron, 9:883-896, 1992). NGF and BDNF were obtained from Peprotech (Rocky Hill, NJ).

### Immunoprecipitation and Immunoblotting

Immunoprecipitations and immunoblotting were performed as previously described (Kaplan et al., Science, 252:554-558, 1991). Briefly, after two washes with ice-cold Tris-buffered saline, cells were lysed in I ml of 1% Nonidet P-40 (NP-40 lysis buffer (20 mM Tris [pH8.0], 137 mM NaCl, 0.5 mM EDTA, 10% glycerol, 1 mM phenylmethylsulphonyl fluoride, 0.15 U/ml aprotinin, 20 µM leupeptin, 1 mM sodium vanadate) at 4°C for 20 min. Insoluble material was removed by centrifugation at 4°C for 10 min at 10,000 x g. Immunoprecipitations were performed for 4-6 hr at 4°C. Precipitates were collected with protein A-Sepharose™, then washed three times with NP-40 lysis buffer and once with water. The immunoprecipitates were boiled in sample buffer (2% SDS, 100mM dithiothreitol, 10% glycerol, 0.25% bromophenol blue) for 5 min and electrophoretically fractionated by SDS-PAGE. Protein blots were probed overnight at 4°C with antibody. Antibody labeling of proteins was visualized using horseradish peroxidase-coupled secondary antibody from Boehringer Mannheim Biochemicals (Laval, Quebec) and the ECL chemiluminescence system (Amersham Corp., Arlington Heights, IL).

### Baculovirus Expression of Proteins and SHP/Trk Association Assay

Sf9 insect cells (2x10⁶) were infected with recombinant baculoviruses at a multiplicity of infection of I to 10 and lysed at 48 hr postinfection. Sf9 cells were assayed for the expression of the recombinant proteins by immunoblot analysis, as described above. Recombinant baculoviruses were prepared as previously described (Stephens et al., Neuron, 12:691-705, 1994).

The *in vitro* association assay using baculovirus-expressed proteins was performed as described (Kaplan et al., *supra*). Briefly, Sf9 cells (2 x 10⁶) were infected, harvested, and Iysed in NP-40 lysis buffer. Trk proteins were immunoprecipitated with anti-Trk (203) antibody, and the immunoprecipitates were washed once with lysis buffer containing 1% deoxycholate and 0.1% SDS (RIPA)) and three times with 50 mM Tris (pH 7.4). Trks then were activated (i.e. autophosphorylated) by incubation with 50 mM Tris (pH 7.4), 5 mM MnCl₂, 1 mM ATP and 200 µM orthovanadate. Trk proteins then were washed twice with NP40 lysis buffer and were resuspended in 1 ml of PC12 cell lysate. The immunoprecipitated, activated Trk complexes were incubated with the cell lysate for 3 hr at 4°C, and then washed three times with NP-44 lysis buffer and once with 10 mM Tris (pH 7.4) before immunoblot analysis.

### Adenoviruses and Cell Survival Assay

SHP-2 adenoviruses were prepared as previously described (Slack et al, J. Cell Biol., 135:1085, 1996). Neurons were seeded at 5,000 cells per well, and infected with SHP-2 WT (wild-type), SHP-2 ACT (dominant-activated, due to the mutation of the catalytic cysteine to serine), or Bcl-2 adenovirus as described (Slack et al., *supra*). Cells were cultured for 48 hours, and cell viability was measured by the metabolic conversion of a tetrazolium salt into formazan salt according to the CellTiter 96 Assay Kit (Promega; Madison, WI).

### Genbank Accession Numbers

The Genbank Accession Numbers for SHP-1 and SHP-2 are M77273 and L03535, respectively.

### EXAMPLE II

### Neurotrophins stimulate activation of SHP-1 and SHP-2 in neurons

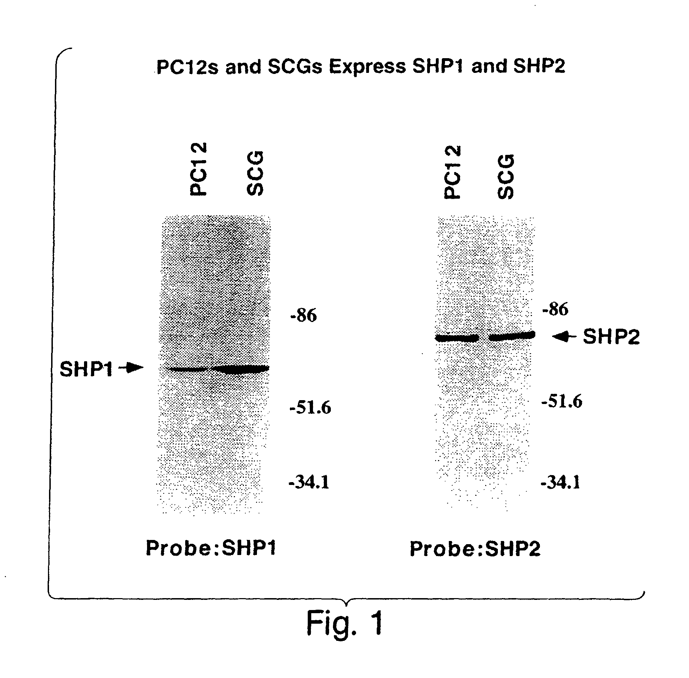

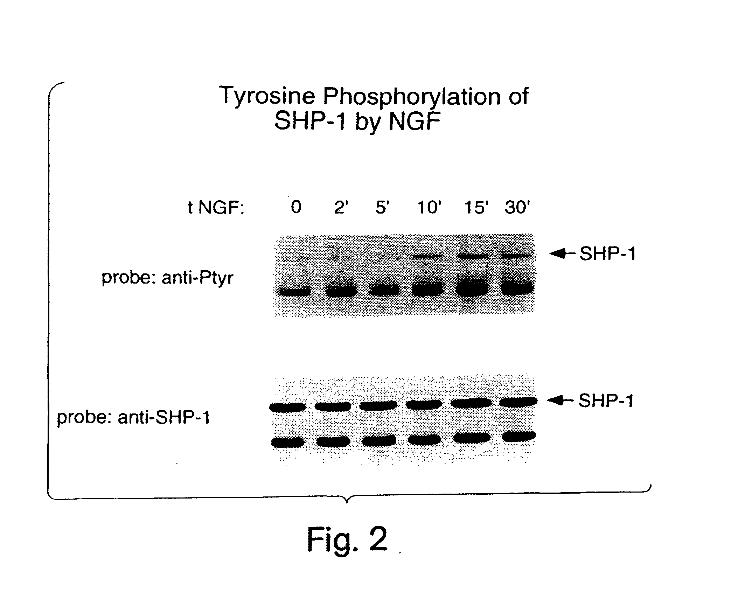

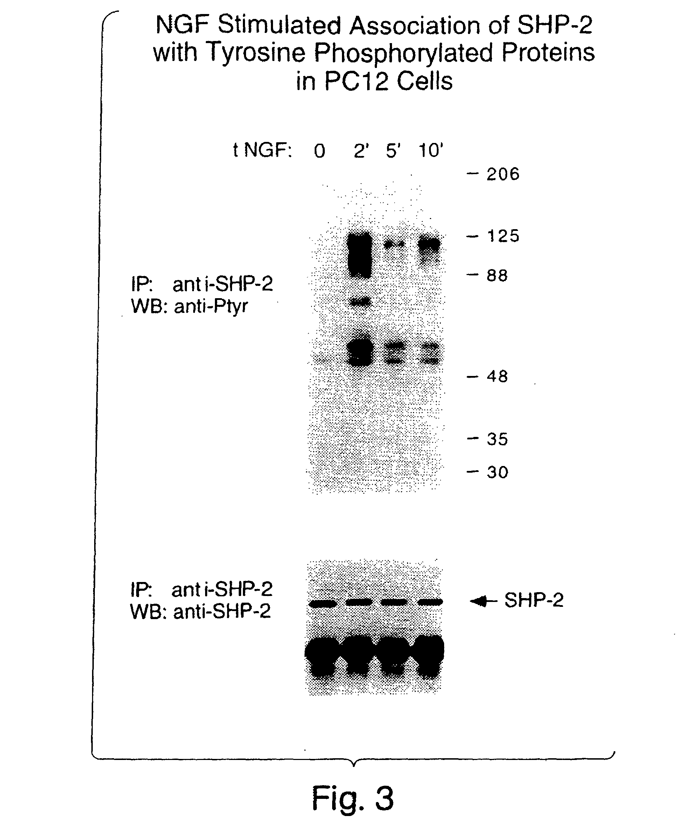

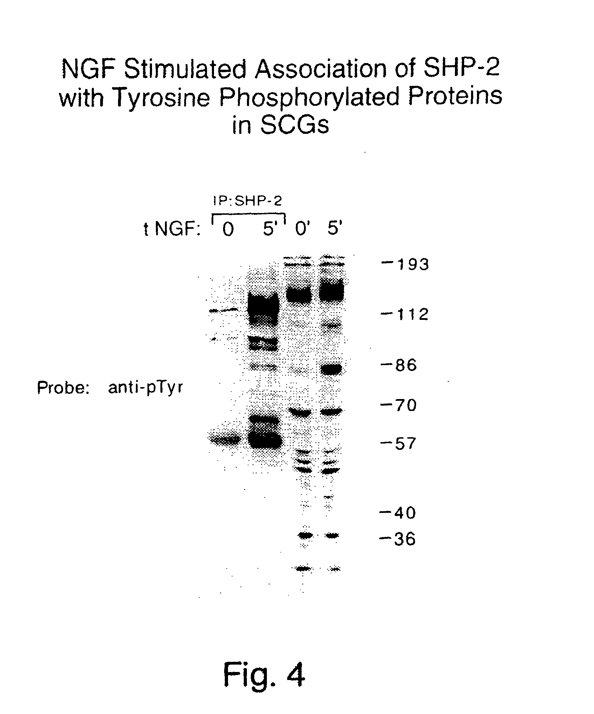

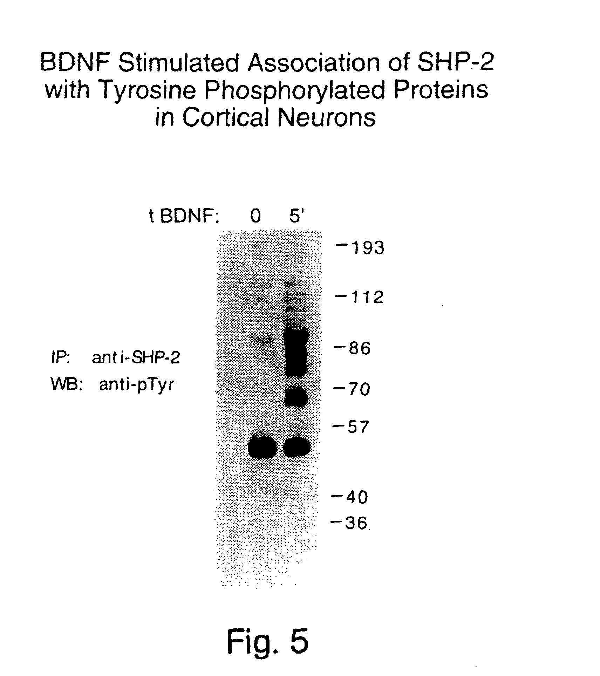

We first asked whether neuronal cells express SHP-1 and SHP-2. Protein (100 µg) from PC12 pheochromocytoma cells (which are known to those skilled in the art as providing a model for sympathetic neuron development) and protein (50 µg) from sympathetic cervical ganglion (SCG) neurons cultured for 5 days was fractionated by SDS-PAGE, transferred to nitrocellulose, and probed either with anti-SHP-1 (Transduction Laboratories) or anti-SHP-2 (Upstate Biotechnology, Inc.) antibody. SHP-1 (Fig. 1, left panel) and SHP-2 (Fig. 1, right panel) protein are present in both PC12 cells and SCG cells. The expression of SHP-1 in neuronal cells is surprising, since previously it was thought that SHP-1 expression is restricted mainly to cells of hematopoietic lineage. Furthermore, NGF stimulates an increase in the tyrosine phosphorylation of SHP-1 in PC12 cells (Fig. 2). SHP-1 phosphorylation levels increase at 10 minutes of neurotrophin treatment and remained elevated at 30 minutes after neurotrophin treatment. Moreover, NGF treatment of PC12 cells stimulates the association of SHP-2 with a number of tyrosine phosphorylated proteins, ranging in size from 50 to 125 kDa (Fig. 3). In addition, in NGF-treated SCG cultures, SHP-2 could be seen to associate with at least five tyrosine phosphorylated proteins, including a protein of approximately 115 kDa (Fig. 4). Furthermore, BDNF cause the association of tyrosine-phosphorylated proteins with SHP-2 in cortical neurons (Fig. 5). In summary, neurotrophins activate SHP-1 and SHP-2 in neurons.

### EXAMPLE III

### Expression of SHP-2 increases cell death in SCG neurons

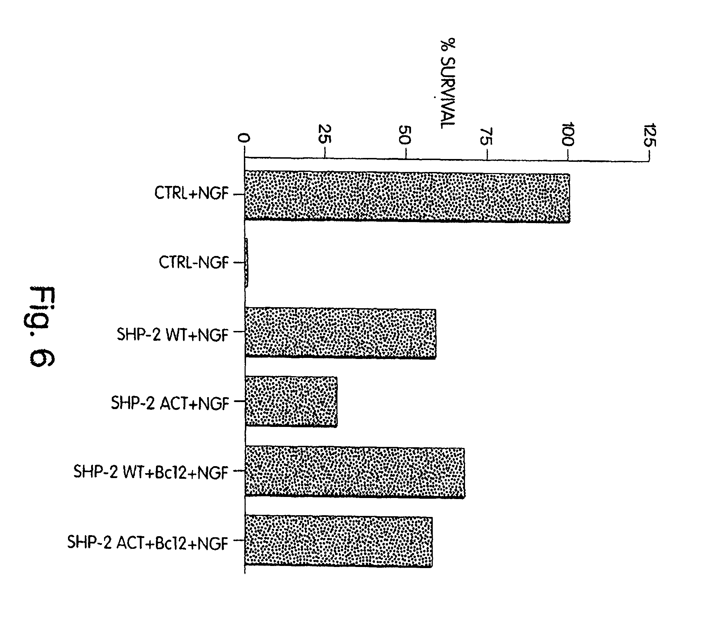

We next asked whether SHP-2, when overexpressed in primary neurons, has the potential to modulate neurotrophin responses. Recombinant adenoviruses expressing wild-type and mutated forms of SHP-2 were generated. Two forms of SHP-2 were selected for expression: wild-type (WT) SHP-2 and dominant-activated (ACT) SHP-2. The dominant-activated form of SHP-2 was constructed by mutating the catalytic cysteine to a serine. Cultured SCG neurons were infected with adenoviruses encoding either wild-type SHP-2 (SHP-2 WT), dominant-activated SHP-2 (SHP-2 ACT) or either of these vimses together with virus encoding Bcl-2. SHP-2 WT and SHP-2 ACT were used at 100 MOI (multiplicity of infection); Bcl-2 adenovirus was used at 50 MOI. Neuronal survival was quantitated 48 hrs post-infection using an assay employing the tetrazolium dye MTT, according to the manufacturer's protocol (Promega). Neuronal survival is expressed as a percentage of the survival of control (non-infected) SCG cultures maintained in 20 ng/ml of NGF. Expression of both WT SHP-2 and ACT SHP-2 markedly decrease neuronal survival over a 48 hour period (Fig. 6). Coexpression of the anti-apoptotic protein Bcl-2 with either of the SHP-2 mutants counteracts the death-inducing effect of SHP-2. Thus, SHP-2 expression is sufficient to cause cell death of neurons. This neuronal cell death is most likely mediated by an apoptotic signal transduction pathway that can be blocked by known apoptosis inhibitors such as Bcl-2.

### EXAMPLE IV

### SHP-1 associates with baculovirus-expressed Trk receptors

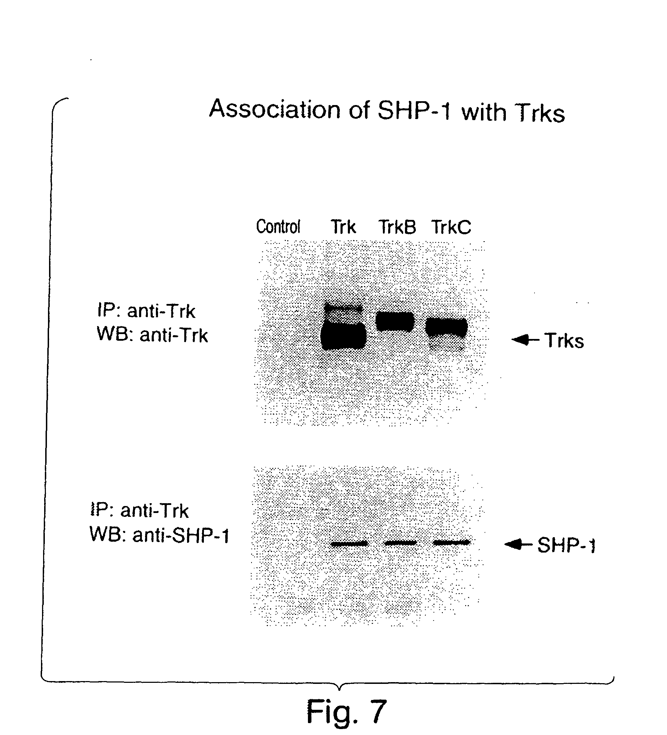

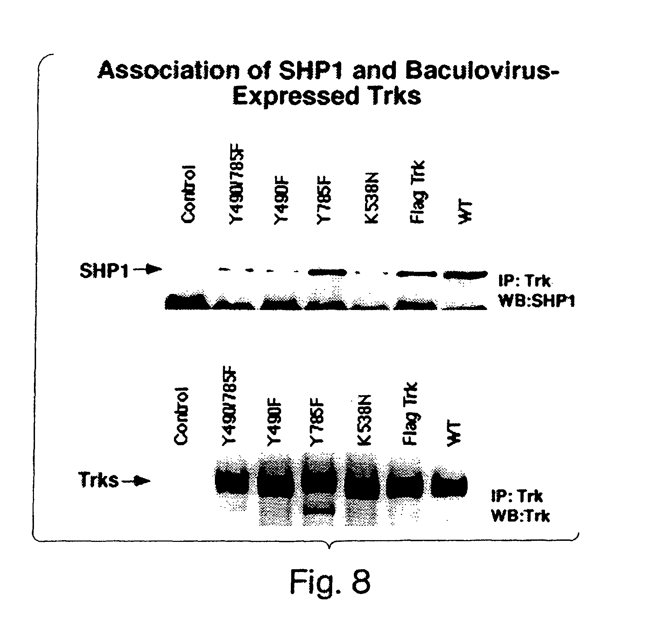

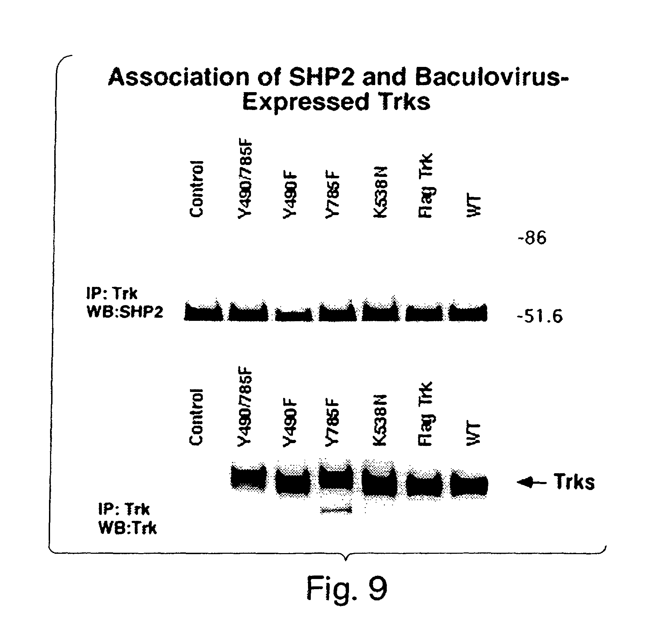

To investigate the mechanism whereby SHP inhibits NGF-induced signaling responses, we first asked whether the SHPs bound to Trk. SHP-1 binds to activated (i.e. autophosphorylated) TrkA, TrkB and TrkC when SHP-1 and Trk are coexpressed in insect cells (Fig. 7) and 3T3 fibroblasts (not shown). We identified the site of SHP-1 association with Trk by coexpressing, in insect cells, SHP-1 with Trks encoding mutations at tyrosines necessary for interactions of Trk with other signaling proteins (Fig. 8). Wild-type and mutant TrkA receptors were expressed in Sf9 cells and immunoprecipitated and activated as described above. The immobilized Trk was incubated with lysates prepared from PC12 cells, washed extensively, electrophoresed, and probed with anti-SHP-1 (Fig. 8, top panel) or anti-Trk (Fig. 8, bottom panel) antibodies. We find that the Y490 of Trk is required for SHP-1 interactions. In contrast, SHP-2, even when overexpressed in insect cells, is unable to bind to Trk (Fig. 9).

### EXAMPLE V

### Dephosphorylation of Trk receptors by SHP-1 and SHP-2

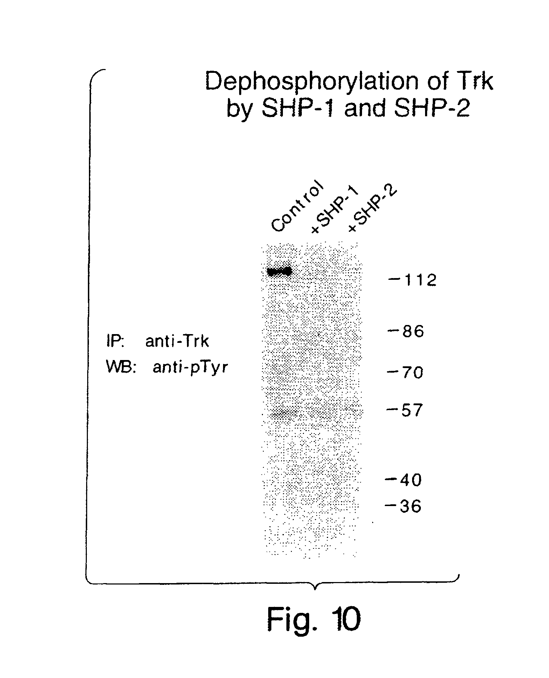

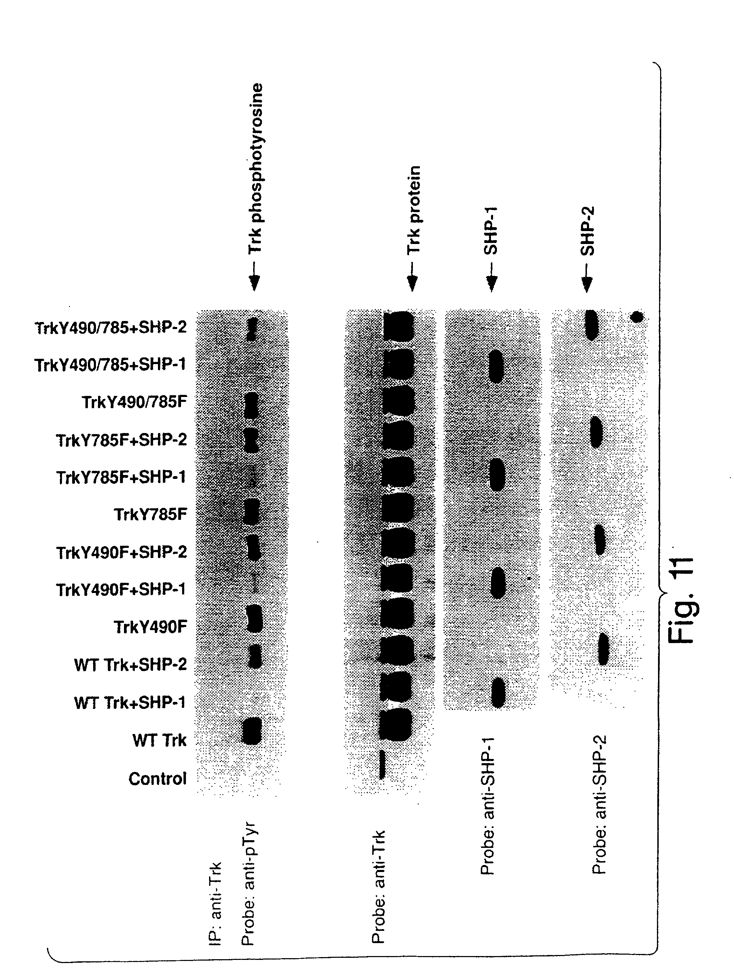

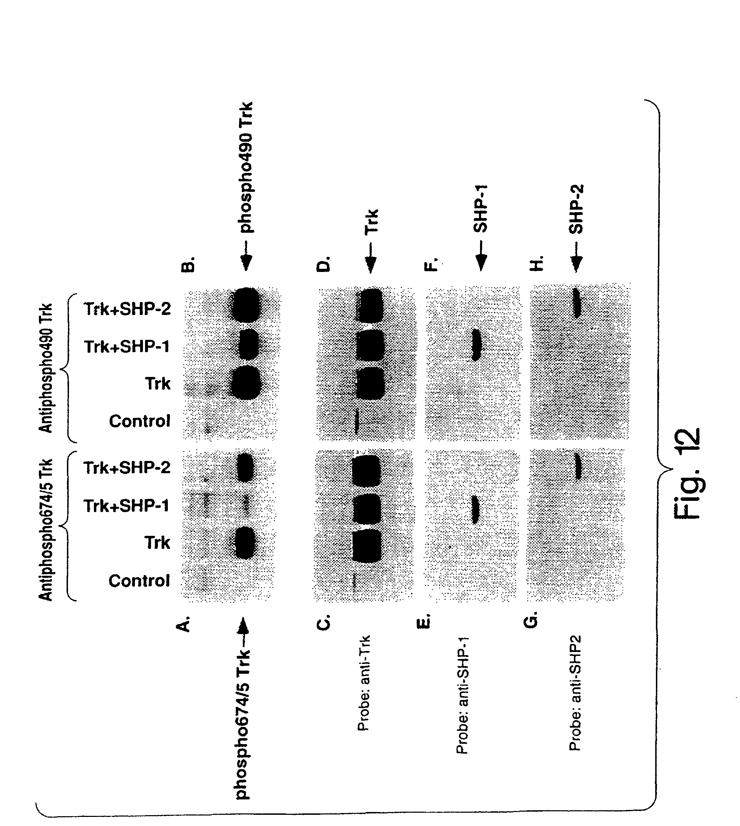

We hypothesized that the suppressive effects of SHP upon NGF-mediated survival might be due to dephosphorylation of the autophosphorylated Trk receptor. Given that SHP-1 can bind to activated Trk and that SHP-2 is activated by Trk (see Example II), we sought to determine whether the SHPs could dephosphorylate TrkA *in vitro* and *in vivo*. Trk, SHP-1, and SHP-2 were expressed in Sf9 cells and isolated by immunoprecipitation with anti-Trk, anti-SHP-1, or anti-SHP-2 antibodies. Immunoprecipitated Trk was autophosphorylated by incubating with 10 mM MnCl₂, 20 mM Tris pH 7.4 and 2.0 µCi/ml [γ-³²P]-ATP(3000 Ci/mmol) for 30 minutes at room temperature. Activated, radiolabeled Trk then was incubated with partially purified SHP-1 or SHP-2 for 20 minutes at 30° C. *In vitro,* both SHP-1 and SHP-2 are able to dephosphorylate Trk to a similar extent (Fig. 10) i.e., approximately 5-fold dephosphorylation. Moreover, coexpression of Trk with SHP-1 or SHP-2 in insect cells results in Trk dephosphorylation. *In vivo*, both SHP-1 and SHP-2 reduce Trk tyrosine phosphorylation, even when the receptors lack tyrosine 490 (Y490F) and/or tyrosine 785 (Y785F) (Figs. 11 and 12).

In order to map the sites of dephosphorylation two approaches were taken. First, we asked whether Trks mutated at tyrosines 490 and 785 could be dephosphorylated by SHP-1 or SHP-2 when coexpressed in insect cells (Fig. 11). SHP-1 extensively dephosphorylates (about 8-fold) all of the Trks, both wild-type and mutated, whereas SHP-2 causes a more modest dephosphorylation (about 2-fold). Hence, SHPs dephosphorylate Trk mutated at of the five major tyrosine autophosphorylation sites. To determine whether the two other major tyrosine phosphorylation sites in Trk, Y674 and 675, are substrates of the SHPs, wild-type TrkA and the SHPs were coexpressed in insect cells, and Trk phosphorylation at Y674, Y675 and Y490 was assessed using phospho-specific anti-Trk antibodies directed at tyrosines 674/675 and 490. SHP-1 completely dephosphorylates Trk (about 10-fold) at the 674/675 sites, while SHP-2 partially dephosphorylates Trk (about 2-fold) at these sites. Tyrosine phosphorylation of the Y490 site also is decreased by SHP-1. These results show that the SHPs modulate Trk phosphorylation and hence, regulate Trk activity. Since SHP-2 expression stimulates sympathetic neuron death (Fig. 6), we conclude that SHP functions by associating with neurotrophin-activated Trk, dephosphorylating the receptor at sites crucial for maintaining Trk activity, and thereby suppressing neuronal survival.

### EXAMPLE VI

### Assays for the identification of compounds that modulate SHP biological activity

Methods of observing changes in SHP phosphorylation, phosphatase activity, and association with Trks and other cellular proteins, gene expression, protein levels, and mRNA levels, as was described above, are exploited in high-throughput assays for the purpose of identifying compounds that modulate SHP activity and neuronal cell death. Such identified compounds may have utility as therapeutic agents in the treatment of neurodegenerative disease and neurological trauma.

### Enzyme-linked immunosorbant assays

Enzyme-linked immunosorbant assays (ELISAs) are easily incorporated into high-throughput screens designed to test large numbers of compounds for their ability to modulate levels of a given protein. When used in the methods of the invention, changes in a given protein level of a sample, relative to a control, reflect changes in SHP biological activity of the cells, cell lysates, or purified or partially-purified molecules within the sample. Protocols for ELISA may be found, for example, in Ausubel et al., *Current Protocols in Molecular Biology*, John Wiley & Sons, New York, NY, 1997. In one embodiment, the so-called "sandwich" ELISA, samples comprising cell lysates or purified molecules treated as described in Examples I-V are loaded onto the wells of microtiter plates coated with "capture" antibodies. Unbound antigen is washed out, and a second antibody, coupled to an agent to allow for detection, is added. Agents allowing detection include alkaline phosphatase (which can be detected following addition of colorimetric substrates such as *p*-nitrophenolphosphate), horseradish peroxidase (which can be detected by chemiluminescent substrates such as ECL, commercially available from Amersham) or fluorescent compounds, such as FITC (which can be detected by fluorescence polarization or time-resolved fluorescence). The amount of antibody binding, and hence the level of a death marker within a lysate sample, is easily quantitated on a microtiter plate reader. It is understood that appropriate controls for each assay are always included as a baseline reference.

Numerous variations of the basic assay may be employed. For example, in order to detect changes in SHP phosphorylation status, duplicate ELISAS are used. The capture antibody for both assays is specific for SHP, and the second (detection) antibody for the second assay is specific for phosphoprotein. Hence, the first ELISA gives a measure of the absolute quantity of SHP present in a sample, whereas the second reveals the degree of SHP phosphorylation of the sample. Duplicate ELISAs also are performed for the appropriate control samples. For example, to test the effect of a compound on SHP phosphorylation, the duplicate ELISAs are performed on samples not treated with the test compound

To detect modulation of SHP phosphatase activity by a test compound, the capture antibody is against SHP, and the detection antibody is against a SHP substrate, for example, Trk. The detection antibody in the first ELISA is against unphosphorylated Trk, to gain a measure of total Trk within the sample, and the detection antibody in the second assay is against phosphoprotein, or phosphoTrk, to reveal the degree of substrate phosphorylation, when compared to a control not treated with the test compound. The first ELISA, in which the detection antibody is for total (i.e., regardless of phosphorylation status) protein, also reveals changes in the association of SHP with its interaction partners, such as Trk.

A positive assay result, that is, identification of a compound that modulates SHP biological activity, is indicated by a change of at least 3- fold in SHP phosphorylation, a change of at least 2-fold in the amount of SHP associated with other proteins, such as phosphoproteins or Trks, or a change of at least 3-fold in SHP phosphatase activity.

High-throughput assays for the purpose of identifying compounds that modulate SHP biological activity can be performed using treated samples of cells, cell lysates, baculovirus lysates, and purified or partially-purified molecules. In addition to the above-described assays, it is understood that other assays to analyze protein phosphorylation status, phosphatase activity, and protein-protein interactions can be employed, using methods well-known by those skilled in the art (for examples, see Ausubel et al., *supra*).

### Interaction trap assays

Two-hybrid methods, and modifications thereof, are used to screen for compounds that modulate the physical interactions of SHP with proteins such as Trk. Such assays also are used to identify novel proteins that interact with SHP, and hence may be naturally occurring regulators of SHP. Regulators of SHP, e.g. proteins that interfere with the interaction between SHP and other proteins (e.g. Trk), also are identified by the use of a three-hybrid system. Such assays are well-known to skilled artisans, and may be found, for example, in Ausubel et al., *supra*.

### PCR assays

To screen for compounds that modulate SHP activity by modulating SHP expression levels, in addition to the immunoprecipitation, Western blotting, and ELISA techniques described above, nucleic acid amplification techniques for assaying gene expression are employed. For example, the polymerase chain reaction (PCR), when coupled to a preceding reverse transcription step (rtPCR), is a commonly used method for detecting vanishingly small quantities of a target mRNA. When performed within the linear range, with an appropriate internal control target (employing, for example, a housekeeping gene such as actin), such quantitative PCR provides an extremely precise and sensitive means of detecting slight modulations in mRNA levels. Moreover, this assay is easily performed in a 96-well format, and hence is easily incorporated into a high-throughput screening assay. Cells are cultured, and treated with test compounds as described in the preceding examples. The cells are then lysed, the mRNA is reverse-transcribed, and the PCR is performed according to commonly used methods, (such as those described in Ausubel et al., *Current Protocols in Molecular Biology*, John Wiley & Sons, New York, NY, 1997), using oligonucleotide primers that specifically hybridize with SHP nucleic acids. Changes in SHP nucleic acid levels of samples exposed to test compounds, relative to control samples, indicate test compounds with the ability to modulate SHP gene expression or mRNA stability.

DNA sequences that are used to make oligonucleotide primers for use in SHP rtPCR assays are found in the GenBank database, according to the accession numbers listed above.

### Cell death assays

Alterations in SHP-induced apoptotic cell death is used to screen for compounds that modulate SHP activity. For example, a SHP (including, but not limited to: SHP-1, SHP-2, or SHP-2 ACT) is overexpressed in neurons, as described in Example III, or is overexpressed in other cells known in the art to undergo apoptotic cell death, e.g. fibroblasts or other cell lines. The SHP expression vector may be an adenovirus, as described in Example III, or alternatively, may be a retrovirus or a plasmid vector, for example. These vectors may comprise, for the purpose regulating expression of an operably-linked SHP gene, any one of the many gene promoters known and commonly employed by skilled artisans. Such promoters may be constitutively active, e.g., the SV40 promoter, or inducible, e.g. the metallothionein promoter. Cell death is measured by various assays, including, but not limited to: MTT assays, annexin V assays, TUNEL assays, and propidium iodide assays. A compound considered to possess the ability to modulate the level of SHP-induced cell death is one that induces a change of at least 2-fold in the level of SHP-induced cell death, compared to a sample not treated with the compound.

### Non-neuronal cell systems for assaying SHP biological activity

As described in previous Examples, assays employing cell types other than neurons may be used to screen for compounds that modulate SHP biological activity. These include: protein-protein interaction assays, which may be performed in insect cells, yeast cells, fibroblasts, and other types of cells, for example; cell death assays, which may be performed in any type of cell which undergoes apoptotic cell death, dephosphorylation assays, which may be performed in any cell type, as well as *in vitro*, as described, for example, in Ausubel et al., *supra*.

## Claims

1. A method of identifying a compound that modulates neuronal cell death, said method comprising the steps of
(a) providing a cell;
(b) overexpressing a SHP polypeptide in said cell;
(c) contacting said cell with a test compound; and
(d) determining the level of SHP biological activity in said cell by measuring SHP phosphorylation, SHP phosphatase activity, SHP interaction with Trk cellular protein, SHP gene expression, SHP protein levels or SHP mRNA levels,
wherein an alteration in said level, relative to a cell not contacted with said test compound, identifies said test compound as a compound that modulates neuronal cell death.

2. The method of claim 1, wherein said cell is induced to express said SHP polypeptide and said cell is contacted with said test compound before said SHP polypeptide is overexpressed in said cell.

3. The method of claim 1, wherein said cell is genetically engineered to express said SHP polypeptide.

4. The method of claim 1, wherein said cell is contacted with said test compound after induction of said cell to express increased levels of said SHP polypeptide relative to said cell prior to said induction.

5. The method of claim 1, wherein said SHP polypeptide is a SHP-2 polypeptide, wherein the catalytic cysteine has been mutated to serine.

6. The method of claim 1, wherein said SHP palypeptide is a SHP-1 polypeptide.

7. The method of claim 1, wherein said SHP polypeptide is a SHP-2 polypeptide.

8. The method of claim 1, wherein said activity is SHP phosphorylation.

9. The method of claim 1, wherein said activity is SHP polypeptide levels.

10. The method of claim 1, wherein said alteration in SHP biological activity is a decrease.

11. The method of claim 1, wherein said cell is a non-neuronal cell.

12. A method of identifying a compound that modulates neuronal cell death, said method comprising the steps of:
(a) contacting a cell expressing a recombinant SHP-1 or SHP-2 polypeptide with a test compound; and
(b) determining the level of SHP biological activity by measuring SHP phosphorylation, SHP phosphatase activity, SHP interaction with Trk cellular protein, SHP gene expression, SHP protein levels or SHP mRNA levels,
wherein an alteration in said level, relative to a cell not contacted with said compound, identifies said test compound as a compound that modulates neuronal cell death.

## Patentansprüche

1. Verfahren zur Identifizierung einer Verbindung die neuronalen Zelltod moduliert, dieses Verfahren umfasst die Schritte:
a) Zur-Verfügung-Stellen einer Zelle;
b) Überexprimieren eines SHP Polypeptids in der Zelle;
c) In-Kontakt-Bringen der Zelle mit einer Testverbindung; und
d) Bestimmen des Niveau der biologischen SHP- Aktivität in dieser Zelle durch Messen der SHP- Phosphorylierung, SHP- Phosphatase-Aktivität, SHP- Interaktion mit zellulärem Trk-Protein, SHP- Genexpression, SHP- Proteinspiegel oder SHP- mRNA- Spiegel, wobei eine Veränderung in dem Niveau, im Verhältnis zu einer Zelle, die nicht mit der Testverbindung in Kontakt gebracht wurde, die Testverbindung als eine Verbindung, die neuronalen Zelltod moduliert, identifiziert.

2. Verfahren gemäß Anspruch 1, wobei die Zelle induziert wird um das SHP- Polypeptid zu exprimieren und die Zelle mit der Testverbindung in Kontakt gebracht wird bevor das SHP Polypeptid in der Zelle überexprimiert wird.

3. Verfahren gemäß Anspruch 1, wobei die Zelle genetisch konstruiert ist, um das SHP-Polypeptid zu exprimieren.

4. Verfahren gemäß Anspruch 1, wobei die Zelle mit der Testverbindung, nach Induktion der Zelle zur Expression gesteigerter Spiegel des SHP- Polypeptids im Verhältnis zu Zellen vor dieser Induktion, in Kontakt gebracht wird,.

5. Verfahren gemäß Anspruch 1, wobei das SHP Polypeptid ein SHP-2- Polypeptid ist, wobei das katalytische Cystein zu Serin mutiert wurde.

6. Verfahren gemäß Anspruch 1, wobei das SHP Polypeptid ein SHP-1- Polypeptid ist.

7. Verfahren gemäß Anspruch 1, wobei das SHP Polypeptid ein SHP-2- Polypeptid ist.

8. Verfahren gemäß Anspruch 1, wobei die Aktivität SHP- Phosphorylierung ist.

9. Verfahren gemäß Anspruch 1, wobei die Aktivität SHP- Polypeptid- Spiegel ist.

10. Verfahren gemäß Anspruch 1, wobei die Veränderung in der biologischen SHP-Aktivität eine Verringerung ist.

11. Verfahren gemäß Anspruch 1, wobei die Zelle eine nicht-neuronale Zelle ist.

12. Verfahren zur Identifizierung einer Verbindung die neuronalen Zelltod moduliert, dieses Verfahren umfasst die Schritte:
a) In-Kontakt-Bringen einer Zelle, die ein rekombinantes SHP-1 oder SHP-2- Polypeptid exprimiert mit einer Testverbindung; und
b) Bestimmen des Niveaus der biologischen SHP-Aktivität durch Messen der SHP-Phosphorylierung, SHP- Phosphatase-Aktivität, SHP- Interaktion mit zellulärem Trk-Protein, SHP Genexpression, SHP-Proteinspiegel oder SHP- mRNA- Spiegel, wobei eine Veränderung in dem Niveau, im Verhältnis zu einer Zelle, die nicht mit der Testverbindung in Kontakt gebracht wurde, die Testverbindung als eine Verbindung, die neuronalen Zelltod moduliert, identifiziert.

## Revendications

1. Méthode d'identification d'un composé qui module la mort des cellules neuronales, ladite méthode comprenant les étapes consistant :
(a) à fournir une cellule ;
(b) à surexprimer un polypeptide SHP dans ladite cellule ;
(c) à mettre en contact ladite cellule avec un composé à tester ; et
(d) à déterminer le niveau d'activité biologique de SHP dans ladite cellule en mesurant la phosphorylation par SHP, l'activité phosphatase de SHP, l'interaction de SHP avec la protéine cellulaire Trk, l'expression du gène de SHP, les teneurs en protéine SHP ou les teneurs en ARNm de SHP, dans laquelle une altération dudit niveau, par rapport à celui d'une cellule qui n'est pas en contact avec ledit composé à tester, identifie ledit composé comme un composé qui module la mort des cellules neuronales.

2. Méthode de la revendication 1, dans laquelle ladite cellule est induite à exprimer ledit polypeptide SHP et ladite cellule est mise en contact avec ledit composé à tester avant que ledit polypeptide SHP ne soit surexprimé dans ladite cellule.

3. Méthode de la revendication 1, dans laquelle ladite cellule est génétiquement modifiée pour exprimer ledit polypeptide SHP.

4. Méthode de la revendication 1, dans laquelle ladite cellule est mise en contact avec ledit composé à tester après induction de ladite cellule pour l'expression de niveaux augmentés dudit polypeptide SHP par rapport à ladite cellule avant ladite induction.

5. Méthode de la revendication 1, dans laquelle ledit polypeptide SHP est le polypeptide SHP-2, dans lequel la cystéine catalytique a été mutée en sérine.

6. Méthode de la revendication 1, dans laquelle ledit polypeptide SHP est un polypeptide SHP-1.

7. Méthode de la revendication 1, dans laquelle ledit polypeptide SHP est un polypeptide SHP-2.

8. Méthode de la revendication 1, dans laquelle ladite activité est la phosphorylation par SHP.

9. Méthode de la revendication 1, dans laquelle ladite activité consiste en des teneurs en polypeptide SHP.

10. Méthode de la revendication 1, dans laquelle ladite altération dans l'activité biologique de SHP consiste en une diminution.

11. Méthode de la revendication 1, dans laquelle ladite cellule est une cellule non neuronale.

12. Méthode d'identification d'un composé qui module la mort des cellules neuronales, ladite méthode comprenant les étapes consistant :
(a) à mettre en contact une cellule exprimant un polypeptide SHP-1 ou SHP-2 recombiné avec un composé à testér ; et
(b) à déterminer le niveau d'activité biologique de SHP dans ladite cellule en mesurant la phosphorylation par SHP, l'activité phosphatase de SHP, l'interaction de SHP avec la protéine cellulaire Trk, l'expression du gène de SHP, les teneurs en protéine SHP ou les teneurs en ARNm de SHP, dans laquelle une altération dudit niveau, par rapport à celui d'une cellule qui n'est pas en contact avec ledit composé, identifie ledit composé à tester comme un composé qui module la mort des cellules neuronales.
